# EUROPEAN PATENT APPLICATION

(11) **EP 3 903 798 A1**
(43) Date of publication of application: **03.11.2021**
(21) Application number: 20171456.5
(22) Date of filing: 27.04.2020
(51) Int. Cl.: A61K 36/232, A61K 36/258, A61K 36/286, A61P 19/02, A61P 19/04, A61P 19/08, A61P 19/10, A23L 33/105

(54) **BONE-STRENGTHENING PILL (BSP) AS A DIETARY SUPPLEMENT TO IMPROVE BLOOD CIRCULATION AND STRENGTHEN BONE AND MUSCLE**

(71) Applicant: Wang, Lulin, 10027 Beijing (CN)
(72) Inventor: Wang, Lulin, 10027 Beijing (CN)
(74) Representative: Zaboliene, Reda

(57) **Abstract**

A Bone-Strengthening Pill (BSP) as a dietary supplement to improve blood circulation and strengthen bone and muscle is characterized in that it is made from the following raw materials: Angelica Sinensis, Pseudo-ginseng and Carthamus Tinctorius L.. BSP has the following beneficial effects: promoting blood circulation, dredging microcirculatory disturbance, significantly improving the level of human health, improving blood running in bone tissue and muscle tissue, and preventing or repairing microscopic damage of bone, cartilage tissue and muscle tissue. Especially, BSP has obvious inhibition or reversal function for osteoporosis, fibrous dysplasia, osteonecrosis, osteochondritis, nonunion, bone tuberculosis, osteomyelitis, bone trauma, osteoarthritis, rheumatoid arthritis, bone cyst and other injuries difficult to heal. BSP can be taken for a long term as it is free of toxic and side effects. BSP applies to all kinds of populations with physical weakness, and lesion signs or symptoms in muscle or bone tissue.

## Description

### FIELD OF THE INVENTION

The present invention relates to a dietary supplement which is made from plants as the main raw materials and has the functions of promoting blood circulation, invigorating health, repairing body damage, especially improving human microcirculation, preventing and repairing degeneration to bone & cartilage tissue and muscle tissue.

### DESCRIPTION OF THE RELATED ART

Blood circulation is the basis for the energy supplement and metabolism of a human body, and a variety of diseases will occur in case of poor blood circulation. Microcirculation, in particular, replenishes the body's material needs through the exchange of substances between blood and tissue fluids. Under normal circumstances, the blood flow of microcirculation is adapted to the metabolic level of tissues and organs, which can ensure the blood flow of tissues and organs and regulate the amount of blood returning to heart. If a microcirculation disorder occurs, it will directly affect the physiological functions of various organs. Microcirculatory disorders can take place in many forms, but more common manifestations mainly include physical weakness and decay of muscle or bone tissue. Among them, physical weakness is mostly manifested as general malaise, shortness of breath, atony, action difficulty, insomnia, irritability, frequent colds, etc., and is mostly accompanied by various degenerative processes. The decay of muscle tissue is often manifested as muscle relaxation, frequent sores, unhealed trauma, etc. The decay of bone tissue is mostly manifested as osteoporosis, susceptibility to fracture, chondritis, osteomyelitis, bone tuberculosis, bone cyst and other kinds of inflammation, especially the weight-bearing parts such as femoral head, which are easily damaged or necrotic due to insufficient blood supply.

In the past, body decay caused by blood circulation disorders, especially the inflammation and injury of bone and muscle tissues, were treated in accordance with pathological changes, and medication or surgical treatment was often used. The duration of medication is long, with an uncertain curative effect. Many medicines should not be taken for a long time as they have toxic and side effects. Surgical treatment usually results in larger trauma, with a high cost, and mostly at the cost of local tissue and function loss. Therefore, it is best to prevent or repair the above symptom of body decay, especially the damage to bone tissue and muscle tissue, by dredging the blood flowing pathway and eliminating the microcirculation obstacle before or at the beginning of its discovery. Obviously, to achieve this, it is necessary to change the existing treatment philosophy and practice, and also to achieve the purpose of promoting blood circulation and removing blood stasis, preventing and repairing micro-obstacles through dietary supplements in daily life. Dietary supplements that can realize this purpose are scarce.

### SUMMARY OF THE INVENTION

The purpose of the present invention is to provide a dietary supplement which can dredge blood circulation path, promote microcirculation, prevent or repair microcirculation disturbance, especially strengthen physique, improve and reverse degeneration of bone and cartilage tissue and muscle tissue, supplement the nutrients needed to maintain body movement, safeguard the health, normal metabolism and physiological function of bones and muscles, for a human body; and can be taken for a long time as it has no toxic or side effects.

The purpose above is achieved with the following technical scheme: a Bone-Strengthening Pill (BSP) as a dietary supplement to improve blood circulation, strengthen bone and muscle, and promote the degenerative damage of bone & cartilage and muscle diseases is provided, charicterized in that the dietary supplement is made from the following raw materials: Angelica Sinensis, Panax Notoginseng and Carthamus Tinctorius L..

The proportions (by weight) of the aforesaid raw materials are: 280-320 units of Angelica Sinensis, 55-65 units of Panax Notoginseng, and 12-18 units of Carthamus Tinctorius L..

Lumbricus, Pearl and Borneol are added into the aforesaid raw materials.

The proportions (by weight) of the additional materials are: 80-100 units of Lumbricus, 2-5 units of Pearl and 2-5 units of Borneol.

Codonopsis Pilosula, Rhizoma Atractylodis and Platycodonopsis Grandiflorum are added into the aforesaid raw materials or the additional materials.

180-220 units of Codonopsis Pilosula, 55-65 units of Rhizoma Atractylodis, and 55-65 units of Platycodonopsis Grandiflorum are added into the aforesaid raw materials or the additional materials by weight.

White Peony Root, Ligusticum Wallichii, Polygala Tenuifolia and American Ginseng are added into the aforesaid raw materials or the additional materials.

80-100 units of White Peony Root, 25-35 units of Ligusticum Wallichii, 25-35 units of Polygala Tenuifolia and 2-5 units of American Ginseng are added into the aforesaid raw materials or the additional materials by weight.

Rhizoma Atractylodis Macrocephaiae, Coix Seed, Chinese Yam and Pseudostellaria Heterophylla are added into the aforesaid raw materials or the additional materials.

80-100 units of Rhizoma Atractylodis Macrocephaiae, 28-32 units of Coix Seed, 28-32 units of Chinese Yam, and 2-5 units of Pseudostellaria Heterophylla are added into the aforesaid raw materials or the additional materials by weight.

Astragalus Membranaceus, Psoralen, Polygala Tenuifolia and Radix Glycyrrhizae are added into the aforesaid raw materials or the additional materials.

80-100 units of Astragalus Membranaceus, 28-32 units of Psoralen, 28-32 units of Polygala Tenuifolia and 2-5 units of Radix Glycyrrhizae are added into the aforesaid raw materials or the additional materials by weight.

Achyranthes Bidentata, Sealwort, White Peony Root and Eurycoma Longifolia are added into the aforesaid raw materials or the additional materials.

28-32 units of Achyranthes Bidentata, 28-32 units of Sealwort, 80-100 units of White Peony Root, and 2-5 units of Eurycoma Longifolia are added into the aforesaid raw materials or the additional materials by weight.

80-100 units of Ligusticum Wallichii, 28-32 units of Prepared Rehmannia Glutinosa, 28-32 units of Eucommia Ulmoides and 2-5 units of Rhodiola Rosea are added into the aforesaid raw materials or the additional materials by weight.

The beneficial effect of the invention is as follows: The dietary supplement described hereof has functions to promote blood circulation, dredge microcirculation disturbance, significantly improve the health level, improve blood running of bone and muscle tissue, prevent or repair microscopic damage to bone & cartilage and muscle tissue of a human body, etc., especially to obviously inhibit or reverse osteoporosis, bone fiber hyperplasia, bone necrosis, chondritis, nonunion, bone tuberculosis, osteomyelitis, bone trauma, osteoarthritis, rheumatoid arthritis, bone cyst and other refractory injury, is non-toxic and free of side effects, can be taken for a long term, and is suitable for all kinds of populations with weak physique, muscle or bone tissue with pathological signs or symptoms.

### BRIEF DESCRIPTION OF THE DRAWINGS

FIG. 1 is an experimental image of Embodiment 1;
FIG. 2 is a NMR image of Embodiment 1;
FIG. 3 is a CT image of Embodiment 1;
FIG. 4 is an X-ray image of Embodiment 1;
FIG. 5 is an X-ray image of Embodiment 2;
FIG. 6 is an X-ray image of Embodiment 3;
FIG. 7 is an X-ray image of Embodiment 3;
FIG. 8 is a NMR image of Embodiment 4;
FIG. 9 is a NMR image of Embodiment 4;

### DETAILED DESCRIPTION OF THE PREFERRED EMBODIMENT

Embodiment 1: to produce a dietary supplement that promotes blood circulation and improve bone and muscle. With the experiment name of Bone-Strengthening Pill (BSP), such dietary supplement is made from the following materials: Angelica Sinensis, Panax Notoginseng and Carthamus Tinctorius L.. Some of the above mentioned experiments are for medicinal purposes, but still provide evidence and support for this dietary supplement.

The aforesaid raw materials are described as follows:

Angelica Sinensis, Radix Angelicae Sinensis for Latin name, is a perennial herb of Family Umbelliferae. Its root is one of the most common traditional Chinese medicines.

Panax Notoginseng, Panax pseudoginseng Wall. Var. Notoginseng (Burkill) Hoo et Tseng for Latin name, is a perennial herb in the Panax ginseng genus of Family Araliaceae of Order Umbellales. Its root is used as medicine. In this study, Panax Notoginseng is identified to have a role in improving microcirculation.

Carthamus Tinctorius L., Carthamus tinctorius L. for Latin name, with aliases such as Spinose Carthamus Tinctorius L., is a herbal plant of Genus Carthamus Tinctorius L. of Family Compositae, habitually used as traditional Chinese medicine. The study mainly utilizes Carthamus Tinctorius L.'s functions to promote blood circulation and remove stasis, and to dredge blood microcirculation pathway.

The proportions (by weight) of the aforesaid raw materials are: 280-320 units of Angelica Sinensis, 55-65 units of Panax Notoginseng, and 12-18 units of Carthamus Tinctorius L..

The optimized proportions (by weight) of the aforesaid raw materials are: 300 units of Angelica Sinensis, 60 units of Panax Notoginseng, and 15 units of Carthamus Tinctorius L..

The dietary supplement mentioned hereof may be processed and taken in a variety of ways. This embodiment only gives two examples:

1. All raw materials are powdered, screened with more 80 meshes, and then made into decoction, powder, pill, tablet or capsule. The usage is to orally take 4-10g every time, three times a day, for adults.

All raw materials are crushed, decocted in water, and made into decoction or the decoction is concentrated into decoction, powder, pill, tablet and capsule. The usage is to orally take 2-5g every time, three times a day, for adults.

Safety: The above raw materials are safe and non-toxic. The dosage of this embodiment is lower than the limit specified in the relevant classic medical books, so it can be completely identified as being safe. Nevertheless, standard animal experiments and clinical trials have been conducted in this study to prove that the above compatibility of medicines is safe. The following are the experimental results of three applications:

I. Experiment of weak population: Weakness mainly refers to the physical weakness, body discomfort, weakness after illness, postpartum weakness, weakness, shortness of breath, difficulty in walking, insomnia and forgetfulness, etc. caused by poor blood circulation,

Since 2010, the multi-parameter microcirculation measuring instrument has been used to detect several physically weak persons for 1 6 indexes of three categories, such as loop morphology, blood flow state, and surrounding state of nail fold microcirculation. The dietary supplement was taken by those with abnormal indexes. It deemed as recovery that the indexes are improved, the degenerative symptoms disappear, and no recurrence is observed after one year of follow-up. Recovery number/Total number × 100% = Recovery rate

The experimental situations are shown as follows:

| Microcirculation Index | Total Number (Person) | Recovery Number (Person) | Recovery Rate (%) | Average Duration of Medication (Week) |
|---|---|---|---|---|
| Slight Abnormality | 42 | 35 | 83.3 | 3 |
| Moderate Abnormality | 36 | 29 | 80.6 | 5 |
| Severe Abnormality | 32 | 22 | 68.8 | 8 |

It indicates that this product has a good repair and improvement effect on a variety of microcirculation disturbance.

II. Experiment of bone tissue injury:

1. Animal Experiment: Bone density, osteometrics, fat deposition of femoral head, capillary distribution of femoral head and related biochemical indexes were tested and studied with the castrated rat model of osteoporosis and two-legged rat model of ischemic necrosis of femoral head to evaluate the effect on promoting osteocytes and osteochondrocytes. The experimental results are summarized as follows.

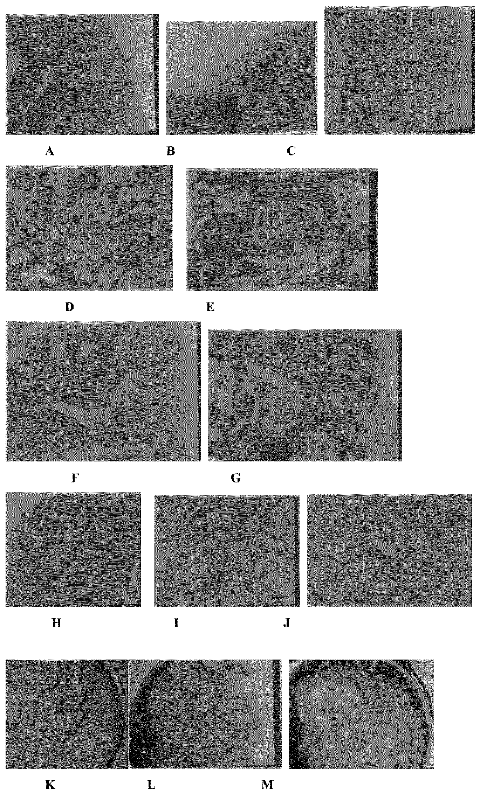

ONFH was induced by prednisone in rats and the occurrence rate is 90%. The surface of the femoral head occurred injuries or defects (Table 1 A Model control). 70% of rats were recovered after taking this product (Table 1 A Experimental group) (FIG. 1A-C). The number of capillaries in the femoral head was reduced and the vessel arcuate cup was disappeared by prednisone. The experimental group was returned to normal after taking this product (FIG. 1K-m).

**Table 1 A The occurrence rate of ONFH model and comparison after taking this product**

| Groups | n | Manifestation types | Occurrence number | Occurrence rate % |
|---|---|---|---|---|
| Model control | 10 | A | 4 | 40 |
| | | B | 5 | 50 |
| | | C | 1 | 10 |
| Experimental group | 10 | A | 0 | 0 |
| | | B | 3 | 30 |
| | | C | 7 | 70 |

| | | | | |
|---|---|---|---|---|
| A: The surface of the femoral head occurred injuries or defects; B: Only osteocytes, chondrocytes and bone marrow cells were changed, and no changes were observed on the surface of the femoral head; C: The surface of the femoral head is smooth with normal osteocytes and chondrocytes. | | | | |

After the inducement of prednisone, the bone cortex and the trabecular bone of rats were fractured and reduced, myelocyte occurred necrosis, the related indicators of bone histomorphometry were significantly reduced, femoral bone trabecula volume (TBV), bone trabecula width (MTPT), cortex of bone volume (CBV), osteoid seam width (MOs W) were significantly lower, lacuna proportion in empty osteocytes (Nocy) was significantly increased, bone mineralization rate (MAR) was significantly decreased. All the indexes of the experimental group were returned to normal after taking this product (Table 1B & FIG. D-G).

**Table 1 B Effect on the bone measurement of the femoral head in model rats after taking this product (⁻× ± SD)**

| **Groups** | **n** | **TBV%** | **MTP** | **TMAR** | **MOSW** |
|---|---|---|---|---|---|
| **Normal control** | **10** | **61.80±3.38** | ****108.08±6.17**** | **1.26±0.15*** | ***1.63±0.11**** |
| **Model control** | **10** | **55.03±5.14** | **93.53±12.28** | **0.66±0.11** | **0.83±0.05** |
| **Experimental group** | **10** | **63.73±6.44** | ****108.20±6.56**** | **1.06±0.17**** | **1.44±0.29**** |

| | | | | | |
|---|---|---|---|---|---|
| Notes: compared with normal control: ****P*<0.01;** compared with model control: ****P*<0.01** | | | | | |

Steroid hormones have been shown to cause ONFH fat accumulation. In the experiment, the number of fat granulosa cells was significantly increased by prednisone, and the experimental group was returned to normal after taking this product (Table 1C) (FIG. 1H-J):

**Table 1 C Effect on osteochondrocytes and osteocyte fat deposition of the femoral head after taking this product**

| **Groups** | **n** | **Average of the test results ±s** | |
|---|---|---|---|
| | | **Fat deposition cell rate %** | **P value** |
| **Normal control** | **10** | **4.74±3.11** | **<0.01** |
| **Model control** | **10** | **16.64±7.42** | - |
| **Experimental group** | **10** | **4.63±2.22** | **<0.01** |

In the evaluation of serological markers associated with bone conversion, the level of tartrate-resistant acid phosphatase TRAP (a biomarker for bone absorption) was increased while the level of ALP (a biomarker for bone formation) was decreased by prednisone. In the experimental group, the above changes were reversed (Table 1D):

**Table 1 D Effects on blood biochemistry of model rats after taking this product**

| **Groups** | **n** | **Test results *X̅* ± S** | |
|---|---|---|---|
| | | **TRAP (B-L)** | **ALP(U)** |
| **Normal control** | **10** | **2.08±0.24^{▲}** | **5.13±0.96** |
| **Model control** | **10** | **2.40±0.33** | **4.57±1.44** |
| **Experimental group** | **10** | **1.47±0.32*** | **6.55±1.34*** |

| | | | |
|---|---|---|---|
| Notes: A **compared with control group P<0.05 *compared with control group P<0.01** | | | |

The experimental results are shown in FIG. 1. In FIG. A, the femoral head of the normal rats has smooth surface and chondrocytes arranging in a columnar manner; in FIG. B, the femoral head of the model rats has surface necrosis and detachment, collapse and coloboma, and loses chondrocytes; in FIG. C, the rats with femoral head necrosis were treated with this product (treatment group) showed that femoral head has smooth surface, is free of coloboma, and recovered chondrocytes in columnar arrangement; in FIG. D, the model rats show crimpiness, disorder and fracture of bone trabecula; in FIG. E, the model rats show the focal necrosis of intramedullary cells of femoral head; in FIG. F., the bone trabecula is rich and morphology is normal in the treatment group; in FIG. G, no necrosis was observed in myeloid cells in the treatment group; in FIG. H, a small number of fat particles were attached to the chondrocyte surface of the normal rat; in FIG. I, a large number of fat particles were contained in the chondrocytes of femoral head of the model rats; in FIG. J, a small number of fat particles were observed in the femoral head of the rats in the experimental group; in FIG. K, the capillaries of femoral head of the normal rats are abundant, with clear edges and interweaving into a network to form an "arcuate cup"; in Fig. L, the capillaries inside the femoral head of the model rats were sparse and the "arcuate cup" disappeared; in FIG. M, the capillaries of the femoral head of the rats in the treatment group were significantly increased, with clear edges, and the "arcuate cup" recovered.

The results showed that this product can increase the minimum bone density (MBD), trabecula bone volume (TBV), mean trabecular plate thickness (MTPT) and cortical bone volume (CBV) of the model rats; can significantly increase the mineral appositional rate (MAR) and mean osteoid seam width (MOSW) of the femoral head of the model rats, decrease the fat deposition of the femoral head and enrich capillary distribution of femoral head; and bone-related biochemical tests showed the increased alkaline phosphatase (ALP) and decreased tartrate-resistant acid phosphatase (TRAP). Conclusion: This product can significantly promote bone growth, increase bone density, restore bone substance and blood supply, inhibit and reverse necrotic osteoblasts and chondrocytes, repair bone and cartilage, and is conducive to the recovery of bone and joint diseases and free of any toxic and side effects.

2. Population Trial: Since 2010, this dietary supplement has been taken by many volunteers who were diagnosed by public hospitals. If the bone tissue decay was controlled or repaired, and no recurrence was observed in the two-year follow-up, it was deemed as recovery. Recovery number/Total number × 100% = Recovery rate. The experimental conditions are shown as follows:

| Type | Total Number (Person) | Recovery Number (Person) | Recovery Rate (%) | Average Duration of Medication (Week) |
|---|---|---|---|---|
| Osteoporosis | 32 | 27 | 84.4 | 20 |
| Osteonecrosis | 36 | 29 | 80.6 | 36 |
| Nonunion of fracture | 32 | 28 | 87.5 | 16 |
| Fibrous dysplasia | 30 | 26 | 86.7 | 34 |
| Bone cyst | 30 | 27 | 90.0 | 30 |
| Chondritis | 34 | 29 | 85.3 | 26 |

It indicates that this product has a good repair and improvement effect on multiple types of bone tissue decay.

### Typical Case:

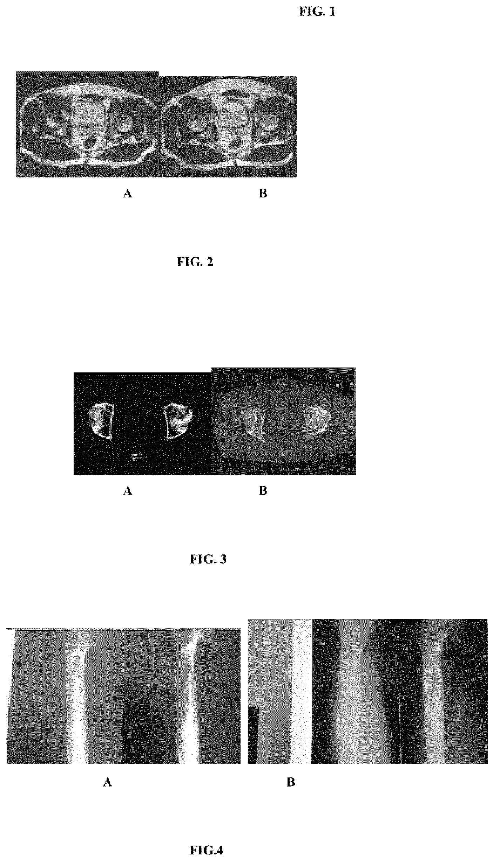

As shown in FIG. 2: A male patient, 37 years old, had been diagnosed with bilateral femoral head necrosis for 3 years, had slight collapse and walking difficulties. He returned to normal work after 12 months of treatment. Before treatment, his MRI image showed abnormal signals of bilateral femoral head, showing large irregularity and uneven signals; abnormal signals of the right femoral head accounted for about 80%, while those of the left for about 60% (A). After treatment, the outline signals of femoral head were uniform, and the abnormal signal area was significantly reduced (B).

As shown in FIG. 3: A male patient, 60 years old, had been diagnosed with bilateral femoral head necrosis for 5 years and was unable to walk. He was cured after 18 months of treatment. Before treatment, his CT image showed that the density of the right femoral head increased unevenly and the necrotic area was about 60%; the left femoral head had fracture, collapse, uneven density and unclear shape, and the necrotic area was about 90% (2-1). After treatment, the density of the right femoral head was uniform and the shape was normal; the shape of the left femoral head was slightly flat, and the density basically returned to being normal (2-2).

III. Experiment of population with muscle tissue decay:
Muscle tissue decay refers to a variety of abnormalities of muscle tissue caused by poor blood circulation and, such as recurrent muscle sores, unhealed skin wounds, osteomyelitis, and unhealed bone tuberculosis fistula or sinus tract, degenerative arthritis and rheumatoid arthritis.

Since 2010, this dietary supplement has been taken by many volunteers who were diagnosed by public hospitals. If the bone tissue decay was controlled or repaired, and no recurrence was observed in the one-year follow-up, it was deemed as recovery. Recovery number/Total number × 100% = Recovery rate.

The experimental results are shown as follows:

| Type | Total Number (Person) | Recovery Number (Person) | Recovery Rate (%) | Average Duration of Treatment (Week) |
|---|---|---|---|---|
| Recurrent sores | 34 | 27 | 79.4 | 16 |
| Unhealed wounds | 36 | 30 | 83.3 | 15 |
| Unhealed fistula and fistulous tract | 36 | 31 | 86.1 | 26 |
| Degenerative arthritis | 30 | 24 | 80.0 | 24 |
| Rheumatoid arthritis | 36 | 23 | 63.9 | 36 |

It indicates that this product has a good repair and improvement effect on various types of muscle tissue decay.

Typical Case: As shown in FIG. 4, a male patient, 36 years old, had been diagnosed with left femoral osteomyelitis for 12 years and undergone 3 fractures and 6 surgeries successively. All the upper and middle section of the femur had skin ulceration, exudation, and necrosis, and unhealed fistulous tract. Once pus flowed, he had to lie in bed. The hospital told him to have an amputation. After 7 months of medication, his skin returned to being normal, his dead bone disappeared, his fistulous tract was healed, and he returned to work.

V. Bone density experiment in the general population: in the process of vast medical experiments, it was found that this product is suitable for the general population as a dietary supplement, which can be taken for a long time to significantly improve the decline of bone density, physical quality, insomnia, forgetfulness, back pain and other common signs of decline in the middle-aged and elderly population. Due to the typical significance of the changes in bone density, a number of comparative experiments have been conducted since 2017. Here is an experimental example:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **12** | **10** | **8** |
| **Experimental group** | **30** | **19** | **9** | **2** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 2: Lumbricus, Pearl and Borneol were added into the raw materials mentioned in Embodiment 1 to form a reinforced formula, with 280-320 units of Angelica Sinensis, 55-65 units of Panax Notoginseng, 12-18 units of Carthamus Tinctorius L., 80-100 units of Lumbricus, 2-5 units of Pearl and 2-5 units of Borneol. 300 units of Angelica Sinensis, 60 units of Panax Notoginseng, 15 units of Carthamus Tinctorius L., 90 units of Lumbricus, 3 units of Pearl and 3 units of Borneol are preferred.

### Introduction to newly added raw materials:

Lumbricus also known as Earthworm, is a representative animal in Class Oligochaeta in Phylum Annelida and one of the important traditional Chinese medicinal materials. It is a nutrient source integrating protein, vitamins, minerals and enzymes.

Peal (Pernulo) is a shiny particle formed in pearl sacs of several pearly shellfishes. It is a nutrient source integrating minerals, amino acids and nutrients.

Borneol, a natural crystalline compound precipitated from the resin of Kapur trees of Dipterocarpaceae plants.

The processing and taking methods, safety, and experimental method of the bone-strengthening pill (BSP) as a dietary supplement are the same as those of Embodiment 1: The following are the experimental results of three applications:

I. The experimental situations of the weak population are as follows:

| Microcirculation Index | Total Number (Person) | Recovery Number (Person) | Recovery Rate (%) | Average Duration of Medication (Week) |
|---|---|---|---|---|
| Slight Abnormality | 46 | 40 | 87.0 | 3 |
| Moderate Abnormality | 36 | 30 | 83.3 | 5 |
| Severe Abnormality | 32 | 25 | 78.1 | 8 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 36 | 31 | 86.1 | 18 |
| Osteonecrosis | 34 | 29 | 85.3 | 32 |
| Nonunion of fracture | 30 | 27 | 90.0 | 14 |
| Fibrous dysplasia | 32 | 28 | 87.5 | 32 |
| Bone cyst | 36 | 32 | 88.9 | 30 |
| Chondritis | 30 | 26 | 86.7 | 25 |

It indicates that this product has a good repair and improvement effect on multiple types of bone tissue decay, which is superior to that of Embodiment 1.

### Typical Case:

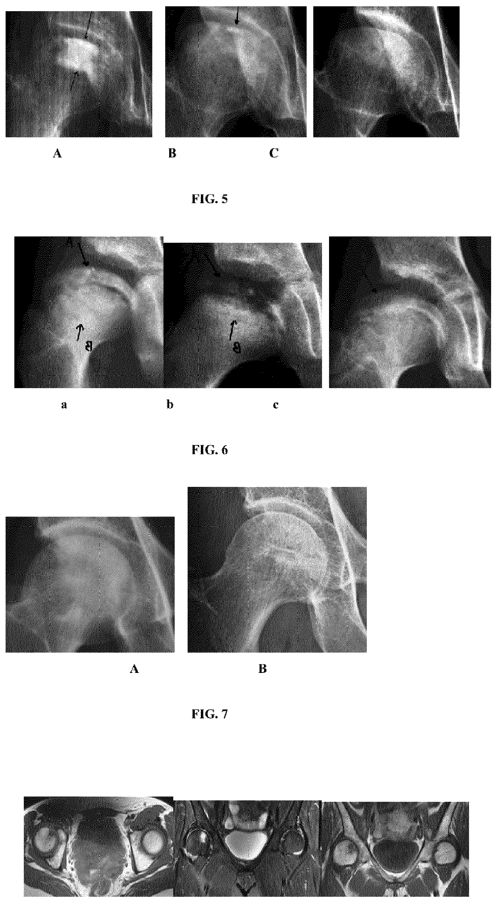

As shown in FIG. 5, a 29-year-old male patient had alcoholism. Before administration, his femoral head had collapsed and deformation and uneven density, and the trabecular bone disappeared (A). There was a significant improvement after treatment (B). After administration, the femoral head looked the same as before, but bone density was uniform, and the patient returned to work (C).

III. The experimental results of the population with muscle tissue decay are shown in the following table:

| Type | Total Number (Person) | Recovery Number (Person) | Overall Response Rate (ORR) (%) | Average Duration of Medication (Week) |
|---|---|---|---|---|
| Recurrent sores | 38 | 32 | 84.2 | 14 |
| Unhealed wounds | 32 | 29 | 90.6 | 14 |
| Unhealed fistula and fistulous tract | 36 | 33 | 91.7 | 24 |
| Degenerative arthritis | 32 | 28 | 87.5 | 22 |
| Rheumatoid arthritis | 36 | 24 | 66.7 | 34 |

It indicates that this product has a good repair and improvement effect on various types of muscle tissue decay.

IV. Bone density experiment in the general population: in the process of vast medical experiments, it was found that this product is suitable for the general population as a dietary supplement, which can be taken for a long time to significantly improve the decline of bone density, physical quality, insomnia, forgetfulness, back pain and other common signs of decline in the middle-aged and elderly population. Due to the typical significance of the changes in bone density, a number of comparative experiments have been conducted since 2017. Here is an experimental example:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **13** | **8** | **9** |
| **Experimental group** | **30** | **21** | **8** | **1** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 3: Codonopsis Pilosula, Rhizoma Atractylodis and Platycodonopsis Grandiflorum are added into the raw materials mentioned in Embodiment 1 to form a reinforced formula, with 280-320 units of Angelica Sinensis, 55-65 units of Panax Notoginseng, 12-18 units of Carthamus Tinctorius L., 180-220 units of Codonopsis Pilosula, 55-65 units of Rhizoma Atractylodis, and 55-65 units of Platycodonopsis Grandiflorum. 300 units of Angelica Sinensis, 60 units of Panax Notoginseng, 15 units of Carthamus Tinctorius L., 200 units of Codonopsis Pilosula, 60 units of Rhizoma Atractylodis, and 60 units of Platycodonopsis Grandiflorum are preferred.

Introduction to newly added raw materials:
Codonopsis Pilosula (*Codonopsis pilosula* (Franch.) Nannf. for scientific name), is a perennial herb of Genus Codonopsis of Family Campanulaceae.

Rhizoma Atractylodis (*Atractylodes Lancea* (Thunb.) DC. for scientific name) is a perennial herb of Genus Atractylodes of Family Compositae.

Platycodonopsis Grandiflorum (*Platycodon grandiflonis* for scientific name), with alias such as Package Flower, Bell Flower and Mitral Flower, is a perennial herb whose root can be used as medicine.

The processing and taking methods, safety, and experimental method of the dietary supplement described herein are the same as those of Embodiment 1:
The following are the experimental results of three applications:

I. The experimental situations of the weak population are as follows:

| Microcirculation Index | Total Number (Person) | Recovery Number (Person) | Recovery Rate (%) | Average Duration of Medication (Week) |
|---|---|---|---|---|
| Slight Abnormality | 44 | 40 | 90.9 | 3 |
| Moderate Abnormality | 36 | 31 | 86.1 | 5 |
| Severe Abnormality | 34 | 29 | 85.3 | 8 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 32 | 28 | 87.5 | 17 |
| Osteonecrosis | 32 | 27 | 84.4 | 30 |
| Nonunion of fracture | 32 | 29 | 90.6 | 13 |
| Fibrous dysplasia | 34 | 29 | 85.3 | 30 |
| Bone cyst | 32 | 28 | 87.5 | 29 |
| Chondritis | 33 | 29 | 87.9 | 25 |

It indicates that this product has a good repair and improvement effect on multiple types of bone tissue decay.

### Typical Case:

As shown in FIG.6, a female patient, 9 years old, has been sick for 2 years and recovered after 16 months of administration . Before administration, her femoral head had coarse surface, increased density, uneven density and epiphyseal density (a). After 7 months of administration, her femoral head had unclear boundaries, decreased density, and uniform epiphyseal density (b). After administration, her femoral head was slightly flat and had smooth surface, clear trabecular bone and uniform density (c).

As shown in FIG. 7, a patient, 45 years old, had a large area of decreased density on the right side according to the X-ray image, and recovered after taking the medicine.

III. The experimental results of the population with muscle tissue decay are shown in the following table:

| Type | Total Number (Person) | Recovery Number (Person) | Overall Response Rate (ORR) (%) | Average Duration of Medication (Week) |
|---|---|---|---|---|
| Recurrent sores | 32 | 28 | 87.5 | 14 |
| Unhealed wounds | 36 | 34 | 94.4 | 14 |
| Unhealed fistula and fistulous tract | 32 | 30 | 93.8 | 22 |
| Degenerative arthritis | 34 | 29 | 85.3 | 22 |
| Rheumatoid arthritis | 36 | 25 | 69.4 | 32 |

It indicates that this product has a good repair and improvement effect on various types of muscle tissue decay, which is superior to that of Embodiment 1.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **11** | **11** | **8** |
| **Experimental group** | **30** | **20** | **9** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 4: Lumbricus, Pearl, Borneol, Codonopsis Pilosula, Rhizoma Atractylodis and Platycodonopsis Grandiflorum are added into the raw materials mentioned in Embodiment 1 to form a reinforced formula, with 280-320 units of Angelica Sinensis, 55-65 units of Panax Notoginseng, 12-18 units of Carthamus Tinctorius L., 80-100 units of Lumbricus, 2-5 units of Pearl, 2-5 units of Borneol, 180-220 units of Codonopsis Pilosula, 55-65 units of Rhizoma Atractylodis, and 55-65 units of Platycodonopsis Grandiflorum.

The optimal proportions (by weight) of the raw materials are as follows: 300 units of Angelica Sinensis, 60 units of Panax Notoginseng, 15 units of Carthamus Tinctorius L., 90 units of Lumbricus, 3 units of Pearl, 3 units of Borneol, 200 units of Codonopsis Pilosula, 60 units of Rhizoma Atractylodis, and 60 units of Platycodonopsis Grandiflorum.

The processing method of the dietary supplement mentioned herein is to powder all raw materials, screen the powder with more 80 meshes, and then made into decoction, powder, pill, tablet or capsule.

The usage of the dietary supplement is to orally take 5-12g every time, three times a day, for adults.

The processing method of the dietary supplement mentioned herein is to crush all raw materials, decoct them in water, and make into decoction or concentrate the decoction into decoction, powder, pill, tablet or capsule.

The usage of the dietary supplement is to orally take 3-6g every time, three times a day, for adults.

The experimental method is the same as that Embodiment 1. The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 43 | 40 | 93.0 | 3 |
| Moderate Abnormality | 33 | 29 | 87.9 | 5 |
| Severe Abnormality | 36 | 31 | 86.1 | 8 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 34 | 30 | 88.2 | 16 |
| Osteonecrosis | 36 | 32 | 88.9 | 30 |
| Nonunion of fracture | 32 | 30 | 93.8 | 12 |
| Fibrous dysplasia | 36 | 32 | 88.9 | 28 |
| Bone cyst | 36 | 33 | 91.7 | 27 |
| Chondritis | 30 | 27 | 90.0 | 20 |

It indicates that this product has a good repair and improvement effect on multiple types of bone tissue decay.

### Typical Case:

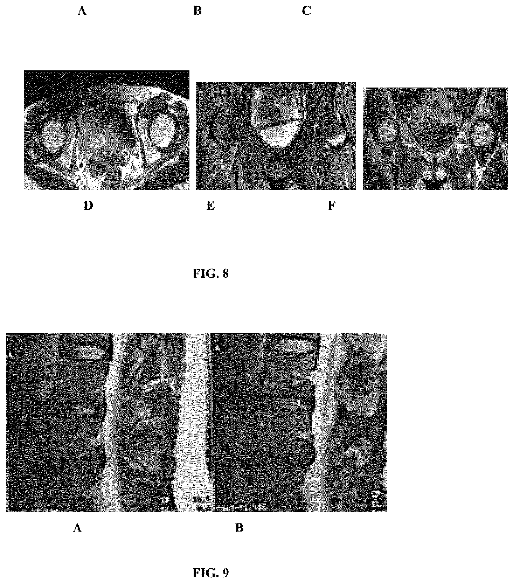

As shown in FIG. 8, a patient, 59 years old, had the decreased signal on the right femoral head and double-line signs (A-C) before treatment. The signal returned to normal after treatment (D-F).

As shown in FIG. 9, a patient, 38 years old, had lumbar sprain, vertebral cartilage avulsion, intervertebral disc herniation, and low back pain with pain in both lower limbs, and was unable to get out of bed or move. He was cured after three months of treatment. Before treatment, the MRI image showed fracture and degeneration of the intervertebral cartilage plates of the lumbar spines #4 and 35, vertebral body avulsion at the ligamentous attachment, intervertebral disc protrusion to the spinal canal, and obvious compression of the nerves in the spinal canal (A). After treatment, the avulsed intervertebral cartilage plate and vertebral body were repaired, and the intervertebral nerve compression and cutting disappeared (B).

III. The experimental results of the population with muscle tissue decay are shown in the following table:

| Type | Total Number (Person) | Recovery Number (Person) | Recovsery Rate(%) | Average Duration of Medication (Week) |
|---|---|---|---|---|
| Recurrent sores | 36 | 33 | 91.7 | 13 |
| Unhealed wounds | 38 | 36 | 94.7 | 12 |
| Unhealed fistula and fistulous tract | 32 | 30 | 93.8 | 20 |
| Degenerative arthritis | 30 | 27 | 90.0 | 20 |
| Rheumatoid arthritis | 36 | 26 | 72.2 | 30 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **12** | **10** | **8** |
| **Experimental group** | **30** | **22** | **8** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 5: White Peony Root, Ligusticum Wallichii, Polygala Tenuifolia and American Ginseng are added into the raw materials Angelica Sinensis, Panax Notoginseng and Carthamus Tinctorius L. to form a reinforced formula, with 280-320 units of Angelica Sinensis, 55-65 units of Panax Notoginseng, 12-18 units of Carthamus Tinctorius L., 80-100 units of White Peony Root, 25-35 units of Ligusticum Wallichii, 25-35 units of Polygala Tenuifolia and 2-5 units of American Ginseng.

300 units of Angelica Sinensis, 60 units of Panax Notoginseng, 15 units of Carthamus Tinctorius L., 90 units of White Peony Root, 30 units of Ligusticum Wallichii, 30 units of Polygala Tenuifolia and 3 units of American Ginseng are preferred.

### Introduction to newly added raw materials:

White Peony Root is dry root of Paeonia tactilora Pall. , a Ranunculaceae plant.

Ligusticum Wallichii (Scientific name:Ligusticum chuanxiong hort) is a herbal plant for traditional Chinese medicine.

Polygala Tenuifolia (Scientific name: Polygala tenuifolia Willd), also known as Fine Grass and Line Tea, is a perennial herb. Traditional Chinese medicine believes that Polygala Tenuifolia has the functions of tranquilizing mind, promoting intelligence, and relieving sputum and swelling. This application is based on the close relationship between human microcirculation and e regulation of thnervous system, so Polygala Tenuifolia is used.

American Ginseng (Scientific name: Panax quinquefolius) is a perennial plant of Genus Panax of Family Araliaceae.

The processing and taking methods, safety and experimental method of the dietary supplement mentioned herein are the same as those of Embodiment 4.

The following are the experimental results of three applications:

I. The experimental situations of the weak population are shown in the following table:

| Microcirculation Index | Total Number (Person) | Recovery Number (Person) | Overall Response Rate (ORR) (%) | Average Duration of Medication (Week) |
|---|---|---|---|---|
| Slight Abnormality | 45 | 42 | 93.3 | 2 |
| Moderate Abnormality | 34 | 30 | 88.2 | 4 |
| Severe Abnormality | 36 | 31 | 86.1 | 7 |

It indicates that this product has a good repair and improvement effect on a variety of microcirculation disturbance, and the time to response is significantly shortened.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 36 | 31 | 86.1 | 14 |
| Osteonecrosis | 32 | 28 | 87.5 | 28 |
| Nonunion of fracture | 36 | 34 | 94.4 | 10 |
| Fibrous dysplasia | 32 | 28 | 87.5 | 26 |
| Bone cyst | 34 | 31 | 91.2 | 26 |
| Chondritis | 36 | 32 | 88.9 | 18 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery (%) | Average Duration of Medication (Week) |
|---|---|---|---|---|
| Recurrent sores | 36 | 31 | 86.1 | 13 |
| Unhealed wounds | 36 | 33 | 91.7 | 6 |
| Unhealed fistula and fistulous tract | 36 | 34 | 94.4 | 18 |
| Degenerative arthritis | 32 | 29 | 90.6 | 18 |
| Rheumatoid arthritis | 36 | 28 | 77.8 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | Maintaining | Invalid |
|---|---|---|---|---|
| **Control group** | **30** | **12** | **11** | **7** |
| **Experimental group** | **30** | **21** | **9** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 6: Lumbricus, Pearl, Borneol, White Peony Root, Ligusticum Wallichii, Polygala Tenuifolia and American Ginseng are added into the raw materials form a reinforced formula, with 280-320 units of Angelica Sinensis, 55-65 units of Panax Notoginseng, 12-18 units of Carthamus Tinctorius L., 80-100 units of Lumbricus, 2-5 units of Pearl, 2-5 units of Borneol, 80-100 units of White Peony Root, 25-35 units of Ligusticum Wallichii, 25-35 units of Polygala Tenuifolia and 2-5 units of American Ginseng. 300 units of Angelica Sinensis, 60 units of Panax Notoginseng, 15 units of Carthamus Tinctorius L., 90 units of Lumbricus, 3 units of Pearl, 3 units of Borneol, 90 units of White Peony Root, 30 units of Ligusticum Wallichii, 30 units of Polygala Tenuifolia and 3 units of American Ginseng are preferred.

The processing and taking methods, safety and experimental method of the dietary supplement described herein are the same as those of Embodiment 4:

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 44 | 42 | 95.5 | 2 |
| Moderate Abnormality | 36 | 33 | 91.7 | 4 |
| Severe Abnormality | 32 | 30 | 93.8 | 7 |

It indicates that this product has a good repair and improvement effect on a variety of microcirculation disturbance, and the time to response is significantly shortened.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 36 | 32 | 88.9 | 12 |
| Osteonecrosis | 36 | 31 | 86.1 | 26 |
| Nonunion of fracture | 32 | 31 | 96.6 | 9 |
| Fibrous dysplasia | 32 | 30 | 93.8 | 24 |
| Bone cyst | 36 | 33 | 91.7 | 24 |
| Chondritis | 32 | 29 | 90.6 | 16 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 36 | 34 | 94.4 | 12 |
| Unhealed wounds | 36 | 35 | 97.2 | 3 |
| Unhealed fistula and fistulous tract | 32 | 31 | 96.9 | 18 |
| Degenerative arthritis | 32 | 29 | 90.6 | 16 |
| Rheumatoid arthritis | 36 | 29 | 80.6 | 26 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **13** | **11** | **6** |
| **Experimental group** | **30** | **23** | **7** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 7: Rhizoma Atractylodis Macrocephaiae, Coix Seed, Chinese Yam and Pseudostellaria Heterophylla are added into the raw materials Angelica Sinensis, Panax Notoginseng, and Carthamus Tinctorius L., to form a reinforced formula, with 280-320 units of Angelica Sinensis, 55-65 units of Panax Notoginseng, 12-18 units of Carthamus Tinctorius L., 80-100 units of Rhizoma Atractylodis Macrocephaiae, 28-32 units of Coix Seed, 28-32 units of Chinese Yam, and 2-5 units of Pseudostellaria Heterophylla.

300 units of Angelica Sinensis, 60 units of Panax Notoginseng, 15 units of Carthamus Tinctorius L., 90 units of Rhizoma Atractylodis Macrocephaiae, 30 units of Coix Seed, 30 units of Chinese Yam, and 3 units of Pseudostellaria Heterophylla are preferred.

### Introduction to newly added raw materials:

Rhizoma Atractylodis Macrocephaiae, Atractylodes macrocephala for Latin name, with aliases such as Raft Thistle, Winter Atractylodes Macrocephala, Yang beam, Poplar Raft, is a perennial herb in Genus Atractylodes in Family Composite. Its rhizome is used as medicine.

Coix Seed (Coix chinensisTod.), also known as Seed of Job's Tears seed and Six Millet Seed, is seed of Coix, a plant of Family Grass.

Chinese Yam, also known as Common Yam Rhizome, Rhizome of Common Yam, Dioscoreae rhizome, Yam, Roachster, and White Yam, is dry rhizome of Chinese Yam, a plant of Family Dioscoreaceae.

Pseudostellaria Heterophylla, Pseudostellaria heterophylla(Miq)Pax for scientific name, belongs to Genus Pseudostellaria in Family Caryophyllaceae (Pseudostellaria heterophylla(Miq)Pax ex Pax et Hoffm). Its root is used as medicine.

The processing and taking methods, safety and experimental method of the dietary supplement mentioned herein are the same as those of Embodiment 4.

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 50 | 48 | 96.0 | 2 |
| Moderate Abnormality | 36 | 34 | 94.4 | 4 |
| Severe Abnormality | 32 | 28 | 87.5 | 7 |

It indicates that this product has a good repair and improvement effect on a variety of microcirculation disturbance and the time to response is significantly shortened.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 36 | 32 | 88.9 | 14 |
| Osteonecrosis | 32 | 28 | 87.5 | 28 |
| Nonunion of fracture | 42 | 40 | 95.2 | 10 |
| Fibrous dysplasia | 36 | 32 | 88.9 | 26 |
| Bone cyst | 34 | 31 | 91.2 | 26 |
| Chondritis | 32 | 29 | 90.6 | 18 |

It indicates that this product has a good repair and improvement effect on multiple types of bone tissue decay, which is superior to that of the previous embodiments.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 36 | 32 | 88.9 | 13 |
| Unhealed wounds | 32 | 30 | 93.8 | 6 |
| Unhealed fistula and fistulous tract | 32 | 31 | 96.9 | 18 |
| Degenerative arthritis | 36 | 33 | 91.7 | 18 |
| Rheumatoid arthritis | 36 | 29 | 80.6 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **14** | **10** | **6** |
| **Experimental group** | **30** | **22** | **8** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 8: Lumbricus, Pearl, Borneol, Rhizoma Atractylodis Macrocephaiae, Coix Seed, Chinese Yam and Pseudostellaria Heterophylla are added to the aforesaid raw materials, form a form a reinforced formula, with 180-220 units of Angelica Sinensis, 55-65 units of Panax Notoginseng, 12-18 units of Carthamus Tinctorius L., 80-100 units of Lumbricus, 2-5 units of Pearl, 2-5 units of Borneol, 80-100 units of Rhizoma Atractylodis Macrocephaiae, 28-32 units of Coix Seed, 28-32 units of Chinese Yam, and 2-5 units of Pseudostellaria Heterophylla.

200 units of Angelica Sinensis, 60 units of Panax Notoginseng, 15 units of Carthamus Tinctorius L., 90 units of Lumbricus, 3 units of Pearl, 3 units of Borneol, 90 units of Rhizoma Atractylodis Macrocephaiae, 30 units of Coix Seed, 30 units of Chinese Yam, and 3 units of Pseudostellaria Heterophylla are preferred.

The processing and taking methods, safety and experimental method of the dietary supplement mentioned herein are the same as those of Embodiment 4. The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 56 | 54 | 96.4 | 2 |
| Moderate Abnormality | 42 | 40 | 95.2 | 4 |
| Severe Abnormality | 32 | 29 | 90.6 | 7 |

It indicates that this product has a good repair and improvement effect on a variety of microcirculation disturbance, and the time to response is significantly shortened.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 36 | 33 | 91.6 | 14 |
| Osteonecrosis | 32 | 29 | 90.6 | 28 |
| Nonunion of fracture | 43 | 42 | 97.7 | 10 |
| Fibrous dysplasia | 36 | 33 | 91.6 | 26 |
| Bone cyst | 36 | 33 | 91.6 | 26 |
| Chondritis | 38 | 35 | 92.1 | 18 |

It indicates that this product has a good repair and improvement effect on multiple types of bone tissue decay, which is superior to that of the previous embodiments.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 38 | 35 | 92.1 | 13 |
| Unhealed wounds | 32 | 31 | 96.9 | 6 |
| Unhealed fistula and fistulous tract | 36 | 35 | 97.2 | 18 |
| Degenerative arthritis | 36 | 33 | 91.7 | 18 |
| Rheumatoid arthritis | 36 | 30 | 83.3 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **13** | **10** | **7** |
| **Experimental group** | **30** | **24** | **6** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 9: Astragalus Membranaceus, Psoralen, Polygala Tenuifolia and Radix Glycyrrhizae are added to the raw materials Angelica Sinensis, Panax Notoginseng and Carthamus Tinctorius L., to form a reinforced formula, with 180-220 units of Angelica Sinensis, 55-65 units of Panax Notoginseng, 12-18 units of Carthamus Tinctorius L., 80-100 units of Astragalus Membranaceus, 28-32 units of Psoralen, 28-32 units of Polygala Tenuifolia and 2-5 units of Radix Glycyrrhizae.

200 units of Angelica Sinensis, 60 units of Panax Notoginseng, 15 units of Carthamus Tinctorius L., 90 units of Astragalus Membranaceus, 30 units of Psoralen, 30 units of Polygala Tenuifolia and 3 units of Radix Glycyrrhizae are preferred

### Introduction to newly added raw materials:

Astragalus Membranaceus (stragalus membranaceus (Fisch.) Bunge.), also known as Radix Astragali, is a perennial herb of Genus Astragalus of Order Rosaceae of Family Leguminosae.

Psoralea (Scientific name: Psoralea corylifoliaLinn.), also known as Fructus Psoraleae, is an annual erect herb of Order Rosaceae of Family Leguminosae. Its fruit is used as medicine.

Radix Glycyrrhizae (Scientific name: Glycyrrhiza uralensis Fisch), also known as Sweet Grass and Sweet Root, is a perennial herb of Order Glycyrrhiza of Family Leguminosae.

The processing and taking methods, safety and experimental method of the dietary supplement mentioned herein are the same as those of Embodiment 4.

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 65 | 61 | 93.8 | 2 |
| Moderate Abnormality | 54 | 49 | 90.7 | 4 |
| Severe Abnormality | 36 | 32 | 88.9 | 7 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 38 | 34 | 89.5 | 14 |
| Osteonecrosis | 36 | 32 | 88.9 | 28 |
| Nonunion of fracture | 39 | 38 | 97.4 | 10 |
| Fibrous dysplasia | 36 | 33 | 91.7 | 26 |
| Bone cyst | 38 | 35 | 92.1 | 26 |
| Chondritis | 36 | 33 | 91.7 | 18 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 46 | 41 | 89.1 | 13 |
| Unhealed wounds | 42 | 39 | 92.9 | 6 |
| Unhealed fistula and fistulous tract | 44 | 42 | 95.5 | 18 |
| Degenerative arthritis | 36 | 33 | 91.7 | 18 |
| Rheumatoid arthritis | 46 | 41 | 89.1 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **12** | **11** | **7** |
| **Experimental group** | **30** | **23** | **6** | **1** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 10: Lumbricus, Pearl, Borneol, Astragalus Membranaceus, Psoralen, Polygala Tenuifolia and Radix Glycyrrhizae are added into the aforesaid raw materials Angelica Sinensis, Panax Notoginseng and Carthamus Tinctorius L., to a form a reinforced formula, with 180-220 units of Angelica Sinensis, 55-65 units of Panax Notoginseng, 12-18 units of Carthamus Tinctorius L., 80-100 units of Lumbricus, 2-5 units of Pearl, 2-5 units of Borneol, 80-100 units of Astragalus Membranaceus, 28-32 units of Psoralen, 28-32 units of Polygala Tenuifolia, and 2-5 units of Radix Glycyrrhizae.

200 units of Angelica Sinensis, 60 units of Panax Notoginseng, 15 units of Carthamus Tinctorius L., 90 units of Lumbricus, 3 units of Pearl, 3 units of Borneol, 90 units of Astragalus Membranaceus, 30 units of Psoralen, 30 units of Polygala Tenuifolia, and 3 units of Radix Glycyrrhizae are preferred.

The processing method of the dietary supplement mentioned herein is to powder all raw materials, screen the powder with more 80 meshes, and then made into decoction, powder, pill, tablet or capsule.

The usage of the dietary supplement is to orally take 5-12g every time, three times a day, for adults.

The processing and taking methods, safety and experimental method of the dietary supplement mentioned herein are the same as those of Embodiment 4.

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 63 | 60 | 95.2 | 2 |
| Moderate Abnormality | 56 | 52 | 92.9 | 4 |
| Severe Abnormality | 38 | 34 | 89.5 | 7 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 41 | 38 | 92.7 | 14 |
| Osteonecrosis | 36 | 33 | 91.7 | 28 |
| Nonunion of fracture | 52 | 51 | 98.1 | 10 |
| Fibrous dysplasia | 36 | 33 | 91.7 | 26 |
| Bone cyst | 40 | 37 | 92.5 | 26 |
| Chondritis | 38 | 35 | 92.1 | 18 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 49 | 45 | 91.8 | 13 |
| Unhealed wounds | 42 | 40 | 95.2 | 6 |
| Unhealed fistula and fistulous tract | 48 | 46 | 95.8 | 18 |
| Degenerative arthritis | 36 | 34 | 94.4 | 18 |
| Rheumatoid arthritis | 48 | 44 | 91.7 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **11** | **12** | **7** |
| **Experimental group** | **30** | **25** | **5** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 11: Achyranthes Bidentata, Sealwort, White Peony Root and Eurycoma Longifolia are added into the aforesaid raw materials Angelica Sinensis, Panax Notoginseng and Carthamus Tinctorius L., to form a reinforced formula, with 180-220 units of Angelica Sinensis, 55-65 units of Panax Notoginseng, 12-18 units of Carthamus Tinctorius L., 28-32 units of Achyranthes Bidentata, 28-32 units of Sealwort, 80-100 units of White Peony Root, and 2-5 units of Eurycoma Longifolia.
200 units of Angelica Sinensis, 60 units of Panax Notoginseng, 15 units of Carthamus Tinctorius L., 30 units of Achyranthes Bidentata, 30 units of Sealwort, 90 units of White Peony Root, and 3 units of Eurycoma Longifolia are preferred.

### Introduction to newly added raw materials:

Achyranthes Bidentata (Latin name: Achyranthes bidentata Blume.), also known as Hyssop, is a perennial herb of Genus Hyssop of Family Amaranthaceae.

Achyranthes Bidentata (Scientific name: Polygonatum sibiricum), also known as Chicken Head Yellow Essence, Yellow Chicken Vegetable, and Pen Tube Vegetable.

Eurycoma Longifolia is a wild shrub in tropical rain forests around the equator in Southeast Asia. It belongs to Genus Euryalium of Family Simarubaceae of Sapindales Order. Its root has various effects.

The processing and taking methods, safety and experimental method of the dietary supplement mentioned herein are the same as those of Embodiment 4. The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 50 | 47 | 94.0 | 2 |
| Moderate Abnormality | 44 | 40 | 90.9 | 4 |
| Severe Abnormality | 38 | 33 | 86.8 | 7 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 44 | 39 | 88.6 | 14 |
| Osteonecrosis | 36 | 32 | 88.9 | 28 |
| Nonunion of fracture | 50 | 48 | 96.0 | 10 |
| Fibrous dysplasia | 36 | 32 | 88.9 | 26 |
| Bone cyst | 39 | 36 | 92.3 | 26 |
| Chondritis | 38 | 35 | 92.1 | 18 |

It indicates that this product has a good repair and improvement effect on various types of bone tissue decay.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 46 | 41 | 89.1 | 13 |
| Unhealed wounds | 44 | 42 | 95.5 | 6 |
| Unhealed fistula and fistulous tract | 36 | 35 | 97.2 | 18 |
| Degenerative arthritis | 42 | 40 | 95.2 | 18 |
| Rheumatoid arthritis | 36 | 30 | 83.3 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **12** | **11** | **7** |
| **Experimental group** | **30** | **23** | **6** | **1** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 12: Lumbricus, Pearl, Borneol, Achyranthes Bidentata, Sealwort, White Peony Root and Eurycoma Longifolia are added into the raw materials Angelica Sinensis, Panax Notoginseng and Carthamus Tinctorius L., to form a reinforced formula, with 180-220 units of Angelica Sinensis, 55-65 units of Panax Notoginseng, 12-18 units of Carthamus Tinctorius L., 80-100 units of Lumbricus, 2-5 units of Pearl, 2-5 units of Borneol, 28-32 units of Achyranthes Bidentata, 28-32 units of Sealwort, 80-100 units of White Peony Root, and 2-5 units of Eurycoma Longifolia.
200 units of Angelica Sinensis, 60 units of Panax Notoginseng, 15 units of Carthamus Tinctorius L., 90 units of Lumbricus, 3 units of Pearl, 3 units of Borneol, 30 units of Achyranthes Bidentata, 30 units of Sealwort, 90 units of White Peony Root, and 3 units of Eurycoma Longifolia are preferred.

The processing and taking methods, safety and experimental method of the dietary supplement mentioned herein are the same as those of Embodiment 4.

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 56 | 54 | 96.4 | 2 |
| Moderate Abnormality | 54 | 51 | 94.4 | 4 |
| Severe Abnormality | 42 | 38 | 90.5 | 7 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 48 | 44 | 91.7 | 14 |
| Osteonecrosis | 38 | 36 | 94.7 | 28 |
| Nonunion of fracture | 62 | 61 | 98.4 | 10 |
| Fibrous dysplasia | 36 | 33 | 91.6 | 26 |
| Bone cyst | 42 | 40 | 95.2 | 26 |
| Chondritis | 38 | 36 | 94.7 | 18 |

It indicates that this product has a good repair and improvement effect on various types of bone tissue decay.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 46 | 43 | 93.4 | 13 |
| Unhealed wounds | 48 | 47 | 97.9 | 6 |
| Unhealed fistula and fistulous tract | 46 | 45 | 97.8 | 18 |
| Degenerative arthritis | 46 | 44 | 95.7 | 18 |
| Rheumatoid arthritis | 38 | 33 | 86.8 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **12** | **11** | **7** |
| **Experimental group** | **30** | **24** | **6** | **0** |

The above experimental results show that the effect or this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 13: Ligusticum Wallichii, Prepared Rehmannia Glutinosa, Eucommia Ulmoides and Rhodiola Rosea are added into the raw materials Angelica Sinensis, Panax Notoginseng and Carthamus Tinctorius L., to form a reinforced formula, with 180-220 units of Angelica Sinensis, 55-65 units of Panax Notoginseng, 12-18 units of Carthamus Tinctorius L., 80-100 units of Ligusticum Wallichii, 28-32 units of Prepared Rehmannia Glutinosa, 28-32 units of Eucommia Ulmoides and 2-5 units of Rhodiola Rosea.
200 units of Angelica Sinensis, 60 units of Panax Notoginseng, 15 units of Carthamus Tinctorius L., 90 units of Ligusticum Wallichii, 30 units of Prepared Rehmannia Glutinosa, 30 units of Eucommia Ulmoides and 3 units of Rhodiola Rosea are preferred.

Introduction to newly added raw materials:
Prepared Rehmannia Glutinosa is the earthnut of Rehmannia Glutinosa (Latin name: Chinese Foxglove), also known as Prepared Rehmannia or Foxglove.

Eucommia Ulmoides (Scientific name: Eucommia ulmoides Oliver), also known as Bakelite, is a plant of Family Eucommiaceae. Its dry bark is used as medicine.

Rhodiola Rosea (Scientific name: Rhodiola rosea L.), with the alias of Rose Rhodiola Rosea and Saul Mapur (Tibetan name), is a perennial herb of Family Crassulaceae of Order Rrosaceae.

The processing and taking methods, safety and experimental method of the dietary supplement mentioned herein are the same as those of Embodiment 4.

The following are the experimental results of three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 55 | 53 | 96.4 | 3 |
| Moderate Abnormality | 44 | 41 | 93.2 | 5 |
| Severe Abnormality | 39 | 34 | 87.2 | 8 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 45 | 41 | 91.1 | 14 |
| Osteonecrosis | 40 | 37 | 92.5 | 28 |
| Nonunion of fracture | 46 | 45 | 97.8 | 10 |
| Fibrous dysplasia | 42 | 39 | 92.9 | 26 |
| Bone cyst | 38 | 36 | 94.7 | 26 |
| Chondritis | 36 | 34 | 94.4 | 18 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 56 | 52 | 92.9 | 13 |
| Unhealed wounds | 54 | 51 | 94.4 | 6 |
| Unhealed fistula and fistulous tract | 36 | 35 | 97.2 | 18 |
| Degenerative arthritis | 42 | 40 | 95.2 | 18 |
| Rheumatoid arthritis | 46 | 40 | 87.0 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **14** | **10** | **6** |
| **Experimental group** | **30** | **25** | **4** | **1** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

Embodiment 14: Lumbricus, Pearl, Borneol, Ligusticum Wallichii, Prepared Rehmannia Glutinosa, Eucommia Ulmoides and Rhodiola Rosea are added into the aforesaid raw materials, to form a reinforced formula with 180-220 units of Angelica Sinensis, 55-65 units of Panax Notoginseng, 12-18 units of Carthamus Tinctorius L., 80-100 units of Lumbricus, 2-5 units of Pearl, 2-5 units of Borneol, 80-100 units of Ligusticum Wallichii, 28-32 units of Prepared Rehmannia Glutinosa, 28-32 units of Eucommia Ulmoides and 2-5 units of Rhodiola Rosea.

200 units of Angelica Sinensis, 60 units of Panax Notoginseng, 15 units of Carthamus Tinctorius L., 90 units of Lumbricus, 3 units of Pearl, 3 units of Borneol, 90 units of Ligusticum Wallichii, 30 units of Prepared Rehmannia Glutinosa, 30 units of Eucommia Ulmoides and 3 units of Rhodiola Rosea are preferred.

The processing and taking methods, safety and experimental method of the dietary supplement mentioned herein are the same as those of Embodiment 4.

The following are the experimental results of the three applications:

I. The experimental situations of the weak population are as follows:

| Microcirculation Index | Total Number (Person) | Recovery Number (Person) | Overall Response Rate (ORR) (%) | Average Duration of Medication (Week) |
|---|---|---|---|---|
| Slight Abnormality | 58 | 57 | 98.3 | 3 |
| Moderate Abnormality | 54 | 52 | 96.3 | 5 |
| Severe Abnormality | 38 | 34 | 89.5 | 8 |

It indicates that this product has a good repair and improvement effect on a variety of microcirculation disturbance.

II. The experimental results of the population with bone tissue injury are shown in the following table:

| Type | Total Number (Person) | Recovery Number (Person) | Recovery Rate (%) | Average Duration of Medication (Week) |
|---|---|---|---|---|
| Osteoporosis | 55 | 52 | 94.5 | 14 |
| Osteonecrosis | 60 | 57 | 95.0 | 28 |
| Nonunion of fracture | 66 | 65 | 98.5 | 10 |
| Fibrous dysplasia | 52 | 49 | 94.2 | 26 |
| Bone cyst | 49 | 47 | 95.9 | 26 |
| Chondritis | 46 | 44 | 95.6 | 18 |

It indicates that this product has a good repair and improvement effect on multiple types of bone tissue decay, which is superior to that of Embodiment 1.

III. The experimental results of the population with muscle tissue decay are shown in the following table:

| Type | Total Number (Person) | Recovery Number (Person) | Recovery Rate (%) | Average Duration of Medication (Week) |
|---|---|---|---|---|
| Recurrent sores | 55 | 52 | 94.5 | 13 |
| Unhealed wounds | 58 | 56 | 96.6 | 6 |
| Unhealed fistula and fistulous tract | 62 | 61 | 98.4 | 18 |
| Degenerative arthritis | 52 | 50 | 96.2 | 18 |
| Rheumatoid arthritis | 48 | 43 | 89.6 | 28 |

It indicates that this product has a good repair and improvement effect on various types of muscle tissue decay.

### IV. Bone density experiment in the general population:

On a voluntary basis, 60 subjects with ages between 50 and 65 were collected and all had the bone density test in a standard hospital with T values between -2.5 -- -1, all of them were mildly low bone density. Among them, 30 patients in the experimental group took orally 4-6g of this product, three times a day. In the control group, 30 people were given calcium carbonate tablets, 1-2g per day. After continuous use for 6 months, recovery referred to the T value was increased between -1 and 1, maintaining referred to the T value did not change, and invalid result referred to the T value was further reduced. The results are as follows:

| **Types** | **n (subjects)** | **Recovery** | **Maintaining** | **Invalid** |
|---|---|---|---|---|
| **Control group** | **30** | **13** | **9** | **8** |
| **Experimental group** | **30** | **26** | **4** | **0** |

The above experimental results show that the effect of this product is relatively significant. Especially in the experimental group, the diet, sleep and defecation of the subjects are in good physical conditions, and they are required to continuously take it. The comprehensive evaluation is significantly better than that in the control group, so as to support the transition of this product from drugs to dietary supplements.

## Claims

1. The Bone-Strengthening Pill (BSP) as a dietary supplement to improve blood circulation and strengthen bone and muscle, being **characterized in that** it is made from the following raw materials: Angelica Sinensis, Panax Notoginseng and Carthamus Tinctorius L..

2. The BSP as a dietary supplement to improve blood circulation and strengthen bone and muscle according to claim 1, being **characterized in that** the proportions (by weight) of the aforesaid raw materials are as follows: 280-320 units of Angelica Sinensis, 55-65 units of Panax Notoginseng, and 12-18 units of Carthamus Tinctorius L..

3. The BSP as a dietary supplement to improve blood circulation and strengthen bone and muscle according to claim 1, being **characterized in** the addition of Lumbricus, Pearl and Borneol into the aforesaid raw materials.

4. The BSP as a dietary supplement to improve blood circulation and strengthen bone and muscle according to claim 2, being **characterized in** the adding proportions (by weight) of the following materials into the aforesaid raw materials: 80-100 units of Lumbricus, 2-5 units of Pearl and 2-5 units of Borneol.

5. The BSP according as a dietary supplement to improve blood circulation and strengthen bone and muscle according to claim 1 or 3, being **characterized in** the addition of Codonopsis Pilosula, Rhizoma Atractylodis and Platycodonopsis Grandiflorum in the aforesaid raw materials.

6. The BSP as a dietary supplement to improve blood circulation and strengthen bone and muscle according to claim 2 or 4, being **characterized in** the adding proportions (by weight) of the following materials into the aforesaid raw materials: 180-220 units of Codonopsis Pilosula, 55-65 units of Rhizoma Atractylodis, and 55-65 units of Platycodonopsis Grandiflorum.

7. The BSP as a dietary supplement to improve blood circulation and strengthen bone and muscle according to claim 1 or 3, being **characterized in** the addition of White Peony Root, Ligusticum Wallichii, Polygala Tenuifolia and American Ginseng into the aforesaid raw materials.

8. The BSP as a dietary supplement to improve blood circulation and strengthen bone and muscle according to claim 2 or 4, being **characterized in** the adding proportions (by weight) of the following materials into the aforesaid raw materials: 80-100 units of White Peony Root, 25-35 units of Ligusticum Wallichii, 25-35 units of Polygala Tenuifolia and 2-5 units of American Ginseng.

9. The BSP as a dietary supplement to improve blood circulation and strengthen bone and muscle according to claim 1 or 3, being **characterized in** the addition of Rhizoma Atractylodis Macrocephaiae, Coix Seed, Chinese Yam and Pseudostellaria Heterophylla into the aforesaid raw materials.

10. The BSP as a dietary supplement to improve blood circulation and strengthen bone and muscle according to claim 2 or 4, being **characterized in** the adding proportions (by weight) of the following materials into the aforesaid raw materials: 80-100 units of Rhizoma Atractylodis Macrocephaiae, 28-32 units of Coix Seed, 28-32 units of Chinese Yam, and 2-5 units of Pseudostellaria Heterophylla.

11. The BSP as a dietary supplement to improve blood circulation and strengthen bone and muscle according to claim 1 or 3, being **characterized in** the addition of Astragalus Membranaceus, Psoralen, Polygala Tenuifolia and Radix Glycyrrhizae into the aforesaid raw materials.

12. The BSP as a dietary supplement to improve blood circulation and strengthen bone and muscle according to claim 2 or 4, being **characterized in** the adding proportions (by weight) of the following materials into the aforesaid raw materials: 80-100 units of Astragalus Membranaceus, 28-32 units of Psoralen, 28-32 units of Polygala Tenuifolia and 2-5 units of Radix Glycyrrhizae.

13. The BSP as a dietary supplement to improve blood circulation and strengthen bone and muscle according to claim 1 or 3, being **characterized in** the addition of Achyranthes Bidentata, Sealwort, White Peony Root and Eurycoma Longifolia into the aforesaid raw materials.

14. The BSP as a dietary supplement to improve blood circulation and strengthen bone and muscle according to claim 2 or 4, being **characterized in** the adding proportions (by weight) of the following materials into the aforesaid raw materials: 28-32 units of Achyranthes Bidentata, 28-32 units of Sealwort, 80-100 units of White Peony Root, and 2-5 units of Eurycoma Longifolia.

15. The BSP as a dietary supplement to improve blood circulation and strengthen bone and muscle according to claim 2 or 4, being **characterized in** the adding proportions (by weight) of the following materials into the aforesaid raw materials: 80-100 units of Ligusticum Wallichii, 28-32 units of Prepared Rehmannia Glutinosa, 28-32 units of Eucommia Ulmoides and 2-5 units of Rhodiola Rosea.
